# EUROPÄISCHE PATENTSCHRIFT

(11) **EP 2 417 477 B1**
(45) Veröffentlichungstag und Bekanntmachung des Hinweises auf die Patenterteilung: **30.08.2017**
(21) Anmeldenummer: 10717045.8
(22) Anmeldetag: 06.04.2010
(51) Int. Cl.: G01T 1/167

(54) **DETEKTORVORRICHTUNG**
DETECTOR DEVICE
DISPOSITIF DE DÉTECTION

(30) Priorität: 07.04.2009 DE 102009002273
(43) Veröffentlichungstag der Anmeldung: 15.02.2012
(73) Patentinhaber: GSI Helmholtzzentrum für Schwerionenforschung GmbH, 64291 Darmstadt (DE)
(72) Erfinder: KRAFT, Gerhard, 64291 Darmstadt (DE)
(86) Internationale Anmeldenummer: PCT/EP2010/002161
(87) Internationale Veröffentlichungsnummer: WO 2010/115608

(56) Entgegenhaltungen:
- WO-A1-2008/009528
- US-A- 3 838 284
- BYUNG-HWI KANG ET AL: "Monte Carlo Design Study of a Gamma Detector System to Locate Distal Dose Falloff in Proton Therapy", IEEE TRANSACTIONS ON NUCLEAR SCIENCE, IEEE SERVICE CENTER, NEW YORK, NY, US, Bd. 56, Nr. 1, 1. Februar 2009 (2009-02-01), Seiten 46-50, XP011251268, ISSN: 0018-9499
- MIN CHUL HEE ET AL: "Development of an Array-Type Prompt Gamma Detection System for the Online Measurement of the Range of the Proton Beam in a Patient: a Monte Carlo Feasibility Study", JOURNAL OF THE KOREAN PHYSICAL SOCIETY, SEOUL, KR, Bd. 52, Nr. 3, 15. März 2008 (2008-03-15), Seiten 888-891, XP002509601, ISSN: 0374-4884
- TESTA E ET AL: "Dose profile monitoring with carbon ions by means of prompt-gamma measurements", NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, Bd. 267, Nr. 6, 1. März 2009 (2009-03-01), Seiten 993-996, XP026067102, ISSN: 0168-583X, DOI: DOI:10.1016/J.NIMB.2009.02.031 [gefunden am 2009-02-11]
- SCROGGS R J ET AL: "A multicrystal gamma-ray spectrometer with time-of-flight rejection of neutron-induced background", IEEE TRANSACTIONS ON NUCLEAR SCIENCE, IEEE SERVICE CENTER, NEW YORK, NY, US, vol. NS-11, no. 1, 1 January 1964 (1964-01-01), pages 365-373, XP002513651, ISSN: 0018-9499, DOI: 10.1109/TNS.1964.4323370
- PAULO CRESPO ET AL: "Direct time-of-flight for quantitative, real-time in-beam PET: a concept and feasibility study; Direct time-of-flight for quantitative, real-time in-beam PET", PHYSICS IN MEDICINE AND BIOLOGY, INSTITUTE OF PHYSICS PUBLISHING, BRISTOL GB, vol. 52, no. 23, 7 December 2007 (2007-12-07), pages 6795-6811, XP020127262, ISSN: 0031-9155

## Beschreibung

Die vorliegende Erfindung betrifft eine Detektorvorrichtung, eine Bestrahlungsvorrichtung und ein Verfahren zur Bestimmung einer Eindringtiefe eines in einem Zielvolumen applizierten Partikelstrahls.

Die Bestrahlung eines Zielvolumens in einem Bestrahlungsvolumen eines Gegenstand mit Ionenstrahlen oder Partikelstrahlen betrifft die Bestrahlung von Materie, insbesondere anorganischer, organischer und biologischer Materialien und wird in den unterschiedlichen Bereichen der Forschung, der Industrie und der Medizintechnik eingesetzt. Das Zielvolumen umfasst dabei insbesondere den Bereich, in dem zur Modifikation des bestrahlten Materials eine vorgegebene Dosis deponiert werden soll; das Bestrahlungsvolumen umfasst insbesondere auch diejenigen Bereiche des Materials, die von Strahlung durchdrungen werden, wobei dann im Zielvolumen die gewünschte Dosis appliziert wird. Unter einem Partikelstrahl oder Ionenstrahl wird insbesondere ein hochenergetischer Strahl aus entweder geladenen Teilchen, beispielsweise Protonen, Kohlenstoffionen oder Ionen anderer Elemente, Pionen oder neutralen Teilchen, beispielsweise Neutronen verstanden. In der nachfolgenden Beschreibung werden die Begriffe Ionenstrahl und Partikelstrahl sowie Teilchenstrahl synonym verwendet. Unter hoher Energie wird insbesondere eine Energie der Partikel im Bereich einiger MeV/amu bis zu einigen GeV/amu (amu: atomare Masseneinheit) verstanden.

Eine zur Durchführung der Bestrahlung geeignete Bestrahlungsvorrichtung weist im Allgemeinen eine den Ionenstrahl erzeugende und formende Beschleunigungseinrichtung auf, wobei der Ionenstrahl zur Bestrahlung über ein Strahltransportsystem in einen Bereich, in dem das Bestrahlungsvolumen angeordnet ist, geführt wird. Ferner umfasst die Bestrahlungsvorrichtung eine Strahlmodifikationseinrichtung, die den Ionenstrahl in seinen Parametern an die Lage und Größe des Zielvolumens anpassen kann. Insbesondere wird die Strahlmodifikationseinrichtung auch als Applikationssystem bezeichnet, das eine Energie, Richtung und Fluenz oder Dosis des lonenstrahls so vorgibt, dass die Dosisverteilung in etwa der Lage und Größe des Zielvolumens entspricht.

Das Bestrahlungsvolumen kann beispielsweise durch eine Nachweiseinrichtung nachgebildet werden, die zur Verifikation eines Bestrahlungsfeldes dient. Das Bestrahlungsvolumen umfasst im Allgemeinen ein Bestrahlungsfeld, das ein Feld mit maximaler Ausdehnung in lateraler Richtung ist, im Allgemeinen in x- und y- Richtung, und senkrecht zur Richtung des lonenstrahls liegt. Die Nachweiseinrichtung kann dabei aus einem Verifikationsfeld oder aus einem so genannten Stack mit mehreren hintereinander angeordneten lateral ausgedehnten Verifikationsfeldern bestehen. Im Bereich der Dosimetrie werden dazu beispielsweise Filme mit einer photographischen Emulsion verwendet. Weiterhin werden Kernspurdetektoren zur Messung der Fluenzverteilung in dem Bestrahlungsfeld eingesetzt. Im Bereich medizinischer Anwendungen wird die Bestrahlung von biologischem Gewebe zum Studium der Wirkung von Partikelstrahlung genutzt, um die Wirkung der Strahlenexposition kosmischer Strahlung im Weltraum abschätzen zu können.

Schließlich kann das Zielvolumen im Bestrahlungsvolumen auch das Volumen eines Tumors in einem Patienten sein. In der Regel wird hierbei das Bestrahlungsvolumen von einem behandelnden Arzt festgelegt und umfasst das eigentliche Zielvolumen, also das Tumorvolumen, sowie einen Sicherheitssaum um das sichtbare Tumorvolumen herum. Ionenstrahlen werden hierbei verwendet, um das Tumorgewebe im Bestrahlungsvolumen zu zerstören.

Bei der Tumortherapie erlauben die besonderen Eigenschaften von Ionenstrahlen, das Tumorgewebe bei minimaler Schädigung des umliegenden gesunden Gewebes einer sehr hohen Dosis zu exponieren. Dies hängt vor allem mit der günstigen Tiefendosisverteilung von Ionenstrahlen zusammen. Beim Eindringen von hochenergetischen Ionenstrahlen in das Material deponieren diese zunächst wenig Energie. Mit zunehmender Tiefe steigt die spezifische Energiedeposition an, erreicht im Bereich einer als Bragg-Peak bezeichneten Verteilungskurve ihr Maximum und fällt danach steil ab. Dadurch lässt sich auch bei tiefer liegenden Tumoren im Tumorgewebe mehr Energie deponieren als im umliegenden gesunden Gewebe. Für schwere Ionen, wie beispielsweise Kohlenstoffionen steigt außerdem die biologische Wirksamkeit im Maximum des Bragg-Peaks an.

Das Zielvolumen wird im Allgemeinen scheibenweise oder schichtweise, in Richtung des Partikelstrahls (z-Richtung) dadurch abgetastet, dass der Bragg-Peak in z-Richtung über das Zielvoumen verschoben oder gescannt wird. Diese Verschiebung des Bragg-Peaks erfolgt in der Regel indem die Energie des Partikelstrahls geändert wird. Das Zielvolumen wird hierbei in so genannte Isoenergieschichten aufgeteilt, wobei die unterschiedlichen Isoenergieschichten jeweils unterschiedliche Energie des Partikelstrahls aufweisen. Die laterale Abtastung des Zielvolumens erfolgt im Allgemeinen indem der Partikelstrahl eine Isoenergieschicht in der x-, y-Ebene bevorzugt Punktweise abtastet. Die x-y-Ebene liegt hierbei im Wesentlichen senkrecht zu der jeweiligen Isoenergieschicht. Ein Abtastpunkt wird im Allgemeinen als Rasterpunkt bezeichnet, sodass das Zielvolumen in Rasterpunkte jeweils mit x,y,z-Koordinatenpunkte aufgeteilt wird, die bevorzugt nacheinander angefahren werden und in denen eine bestimmte Dosis des Partikelstrahls appliziert werden kann.

Dabei kann der zu bestrahlende Körper (insbesondere ein im Inneren des zu bestrahlenden Körpers liegender zu bestrahlender Volumenbereich) statisch/unbewegt oder bewegt vorliegen. Es kann vorkommen, dass sich das Bestrahlungsvolumen im zu bestrahlenden Gegenstand bzw. Teile hiervon, insbesondere das zu bestrahlende Zielvolumen bewegen. Eine Bewegung kann nicht nur translatorisch relativ zu einem äußeren Koordinatensystem erfolgen, sondern auch in Form einer Verschiebung verschiedener Bereiche des zu bestrahlenden Körpers relativ zueinander (einschließlich Verdrehungen und Verformungen) erfolgen.

Um in sich bewegte Körper bestrahlen zu können, werden so genannte vierdimensionale Bestrahlungsverfahren verwendet. Dabei handelt es sich schlussendlich um dreidimensionale Bestrahlungsverfahren, die eine zeitliche Variation aufweisen (mit der Zeit als vierter Dimension). Beispiele für derartige Materialbearbeitungsverfahren gibt es im Bereich der Materialwissenschaften bei der Herstellung hochintegrierter Bauteile (insbesondere Mikroprozessoren und Speicherchips) sowie bei der Herstellung von mikrostrukturierten und nanostrukturierten Mechaniken.

Es können werden Scanning-Verfahren eingesetzt werden. Insbesondere werden drei spezielle Herangehensweisen diskutiert. Hierbei handelt es sich um so genannte Rescanning-Verfahren, Gating-Verfahren sowie Tracking-Verfahren.

Bei Rescanning-Verfahren wird der zu bestrahlende Körper mit einer großen Anzahl von hintereinander erfolgenden Bestrahlungsvorgängen belegt. Bei einem zyklisch wiederkehrenden Bewegungsmuster des bewegten Körpers (bzw. des zu bestrahlenden Zielgebiets) erfolgt dadurch im statistischen Mittel eine ausreichend starke Bestrahlung des Zielvolumens. Problematisch ist jedoch, dass es fast unvermeidbar zu einer relativ hohen Strahlenbelastung von an sich nicht zu bestrahlenden Teilbereichen des Zielkörpers kommt. Darüber hinaus eignen sich Rescanning-Verfahren prinzipbedingt vornehmlich für relativ schnell verlaufende, zyklisch wiederkehrende Bewegungen.

Bei Gating-Verfahren erfolgt eine aktive Bestrahlung des Zielkörpers nur dann, wenn sich der zu bestrahlende Volumenbereich in einem relativ eng begrenzten, definierten Gebiet befindet. Zu anderen Zeitpunkten erfolgt dagegen keine Bestrahlung (in der Regel durch Abschaltung des Teilchenstrahls). Grundsätzlich liefern Gating-Verfahren gute Bestrahlungsergebnisse. Nachteilig ist jedoch die längere Bestrahlungsdauer, die unter anderem zu höheren Kosten führt.

Einen besonders viel versprechenden Ansatz stellen die Tracking-Verfahren dar. Hierbei wird der Bereich, in dem die Strahlung einwirkt, korrespondierend zur Bewegung des zu bestrahlenden Volumenbereichs des Zielkörpers nachgeführt. Tracking-Verfahren vereinen die Vorteile einer präzisen, zielgenauen Behandlung mit relativ kurzen Bestrahlungszeiten.

In dem Artikel "Monte Carlo Design Study of a Gamma Detector System to Locate Distal Dose Falloff in Proton Therapy" von Byung-Hwi Kang und Jong-Won Kim in IEEE Transactions on Nuclear Science, Vol. 56, No. 1, Februar 2009, Seite 46-50 werden Monte-Carlo-Simulationen vorgestellt, die dem Design eines Systems zur Qualitätssicherung bei Protonentherapiesystemen dienen. Bei dem System werden prompte Gammas, die in eine zum einfallenden Protonen-Teilchenstrahl normal stehende Richtung emittiert werden, gemessen und mit dem Ort der distal abfallenden Flanke des Dosiseintrags korreliert.

In dem Artikel "Development of an Array-Type Prompt Gamma Detection System for the Online Measurement of the Range of the Proton Beam in a Patient: a Monte Carlo Feasibility Study" von Chul Hee Min, Jang Guen Park und Chan Hyeong Kim im Journal of the Korean Physical Society, Vol. 53, No. 3, März 2008, Seiten 888-891 wird mithilfe einer Monte-Carlo-Simulation die Einsetzbarkeit eines Detektorssystems, das eine lineare Anordnung von Szintillationsdetektoren und Fotodioden aufweist, zur online-Messung einer Protonenstrahlreichweite überprüft.

In dem Artikel "Dose profile monitoring with carbon ions by means of promptgamma measurements" von E. Testa, M. Bajard, M. Chevallier, D. Dauvergne, F. Le Foulher, N. Freud, J.M. Letang, J.C. Poizat, C. Ray und M. Testa in Nuclear Instruments and Methods in Physics Research B, No. 267, Februar 2009, Seiten 993-996 wird anhand von Messungen aufgezeigt, dass es mithilfe der Messung von prompten Gammas möglich ist, bei einer Ionenstrahltherapie den longitudinalen Dosiseintrag während einer Therapiesitzung in Echtzeit zu kontrollieren.

Der Erfolg einer Tumortherapie auf Grund der Bestrahlung des Zielvolumens, und damit des Tumors hängt weitgehend davon ab, in wieweit der effektive Teil des lonenstrahls nur auf das Zielvolumen konzentriert werden kann.

Deshalb ist es wünschenswert die genaue Lage des lonenstrahls im Zielvolumen eines Gegenstandes während der Bestrahlung desselben möglichst genau zu kennen.

Es ist Aufgabe der vorliegenden Erfindung ein Verfahren und eine Detektorvorrichtung zur Bestimmung einer Position eines in einem Zielvolumen applizierten Partikelstrahl zu schaffen. Es ist ferner die Aufgabe eine Bestrahlungsvorrichtung mit einer derartigen Detektorvorrichtung zu schaffen.

Die Aufgabe wird gelöst durch die Merkmale der unabhängigen Ansprüche. Vorteilhafte Weiterentwicklungen sind jeweils den Merkmalen der abhängigen Ansprüche zu entnehmen.

Die erfindungsgemäße Detektorvorrichtung zur Detektion einer Eindringtiefe eines in einem Zielvolumen applizierten Partikelstrahls weist eine erste Detektionseinrichtung für den Partikelstrahl und eine zweite Detektionseinrichtung auf, wobei die zweite Detektionseinrichtung eingerichtet und ausgelegt ist, Photonen, die im Zielvolumen entstehen, insbesondere Gamma-Quanten, zu erfassen. Dabei ist die zweite Detektionseinrichtung in Strahlrichtung des Partikelstrahls gesehen hinter der ersten Detektionseinrichtung angeordnet. Die erste Detektionseinrichtung ist derart eingerichtet und ausgelegt, dass mittels der ersten Detektionseinrichtung eine Richtung des Partikelstrahls in einer ersten lateralen Dimension sowie in einer zweiten, von der ersten verschiedenen lateralen Dimension bestimmbar ist. Hierdurch können die prompten Gammas, die durch die Abbremsung der Partikel, insbesondere Ionen im Zielvolumen entstehen, nachgewiesen werden. Die prompten Gammas sind Photonen mit einer Energieverteilung von einigen MeV und haben bevorzugt eine kontinuierliche Energieverteilung zwischen etwa 3 und 10 MeV. Die prompten Gammas werden im Wesentlichen isotrop um das Zielvolumen emittiert. Somit kann die mindestens eine Detektionseinrichtung in jeder Winkelrichtung, insbesondere radial um das Zielvolumen angeordnet sein. Bevorzugt ist die Detektionseinrichtung in Strahlrichtung des lonenstrahls gesehen seitlich und/oder unterhalb oder hinter dem Zielvolumen angeordnet. Die Gammas können vorteilhafterweise zum Zeitpunkt der Bestrahlung des Zielvolumens mit dem Partikelstrahl gemessen werden, da die Gammas beim Abbremsprozess der Ionen im Zielvolumen entstehen. Somit können die Photonen zeitgleich mit der Bestrahlung bei der der Partikelstrahl jeweils in einem oder mehreren Rasterpunkten des Zielvolumens appliziert werden kann, erfasst werden. Mit anderen Worten werden die Photonen zum Zeitpunkt gemessen, zu dem der Partikelstrahl im dem Zielvolumen appliziert wird. Die gewonnene Information kann vorteilhafterweise bei der weiteren Durchführung der Bestrahlung und insbesondere zur Steuerung des weiteren Bestrahlungsverlaufes verwendet werden. Hierbei kann beispielsweise eine Energiekorrektur des Partikelstrahls vorgenommen werden, da die Eindringtiefe ermittelt wurde. Es kann ferner beispielsweise, wenn ein Trackingverfahren angewendet wird, eine Nachführung der Energie des Partikelstrahls mit der aus der Detektionseinrichtung gewonnenen Information erfolgen. Handelt es sich um eine Bestrahlung, die Rescanning einsetzt, kann die mittels der Detektionseinrichtung gewonnene Information der Eindringtiefe zur weiteren Durchführung des Bestrahlungsverlaufes, insbesondere der Rescanningparameter verwendet werden.

Mittels der relativen Anordnung der ersten und der zweiten Detektionseinrichtung zueinander kann eine, insbesondere räumliche, Verteilung des Partikelstrahls und/oder dessen Dosisdeposition im Zielvolumen gemessen werden. Es kann eine insbesondere dreidimensionale richtungsaufgelöste Detektion der durch die Bestrahlung des Zielvolumens mit dem Partikelstrahl entstehenden, insbesondere hochenergetischen Photonen oder prompten Gamma-Quanten erfolgen. Bevorzugt ist die erste Detektionseinrichtung zur Messung einer ersten, den Partikelstrahlstrahl charakterisierenden Größe und die zweite Detektionseinrichtung ist zur Messung einer zweiten, den Teilchenstrahl charakterisierenden Größe vorgesehen. Hierbei können in der zweiten Detektionseinrichtung die Photonen bevorzugt gezählt werden und somit eine Fluenz der Partikel im ionenstrahl ermittelt werden. Es kann aber auch eine analoge Messung der Photonen erfolgen.

Bevorzugt kann mit der zweiten Detektionseinrichtung eine Intensität von im Zielvolumen entstehenden Photonen gemessen werden. Die erste Detektionseinrichtung kann bevorzugt derart angeordnet sein, dass diese von dem Partikelstrahl durchstrahlt oder durchquert wird, bevor sie auf das Zielvolumen trifft und in diesem bevorzugt abgebremst und somit deponiert wird. Dadurch kann insbesondere aus der Messung der ersten Detektionseinrichtung eine Verteilung des Partikelstrahls, insbesondere eine Ortslage in x- und/oder y-Richtung relativ zum Zielvolumen gesehen bestimmt werden. Mittels der zweiten Detektionseinrichtung kann der Ort im Zielvolumen in z-Richtung und damit die Eindringtiefe des lonenstrahls ermittelt werden. Da die zweite Detektionseinrichtung bevorzugt die auf diese treffenden Photonen detektiert, kann ermittelt werden, in welcher Isoenergieschicht des Zielvolumens eine bestimmte Dosis deponiert wurde. Dies kann erfolgen, indem die Anzahl der entstehenden Gammas ermittelt, insbesondere gezählt wird. Hieraus kann beispielsweise durch eine Integration der gezählten Partikel, die Dosis der Partikel, die im Zielvolumen bereits deponiert sind, ermittelt werden. Bevorzugt können die Messung mit der ersten Detektionseinrichtung und die Messung mit der zweiten Detektionseinrichtung zeitgleich erfolgen. Insbesondere kann die Messung der Dosis im Wesentlichen zum Entstehungszeitpunkt der Photonen ermittelt werden.

Hierbei ist vorteilhaft, dass mittels der vorstehend beschriebenen Detektorvorrichtung eine örtliche Verteilung des Partikelstrahls und dessen Dosisdeposition in einem Zielvolumen eines Gegenstandes, insbesondere eines Patienten nicht invasiv und vor allem zeitgleich gemessen werden kann. Die während der Bestrahlung - Applikation des Teilchenstrahls- ermittelte Dosisverteilung kann für eine Steuerung der Bestrahlung, insbesondere Applikation verwendet werden. Dies kann vor allem für eine aktive Strahlapplikation, beispielsweise einem Rescanning und/oder Trackingverfahren, wichtig sein, bei dem unter anderem sukzessive einzelne kleine Teilvolumina des Zielvolumens bestrahlt werden.

Bei der Detektorvorrichtung ist die erste Detektionseinrichtung derart eingerichtet und ausgelegt, dass mittels der ersten Detektionseinrichtung eine Richtung des Partikelstrahls in mindestens einer ersten lateralen Dimension bestimmbar ist. Die erste Detektionseinrichtung kann ein erster Detektor sein, der bevorzugt ein Transmissionsdetektor ist und den Partikelstrahl im Wesentlichen unverändert lässt. Die erste Detektionseinrichtung kann insbesondere ein ortsauflösender Detektor sein, beispielsweise kann die erste Detektionseinrichtung eine Drahtkammer oder eine Pixel-Ionisationskammer sein. Bevorzugt ist die erste Detektionseinrichtung ein Detektor mit einer geringen Massenbelegung.

Weiterhin ist die erste Detektionseinrichtung der Detektorvorrichtung derart eingerichtet und ausgelegt, dass mittels der ersten Detektionseinrichtung die Richtung des Partikelstrahls in mindestens einer zweiten, von der ersten verschiedenen lateralen Dimension bestimmbar ist. Hierdurch werden für den Partikelstrahl der auf einen bestimmten als Rasterpunkt oder Voxel bezeichneten Zielbestrahlungspunkt im Zielvolumen die x und/oder y-Koordinate festgelegt. Durch die Bestimmung der Richtung des Partikelstrahls kann somit im Wesentlichen der Ort oder die Position des Partikelstrahls im Zielvolumen bestimmt werden. Insbesondere in Kombination mit Parametern der Strahlführung kann aus der Bestimmung der lateralen (x-, y-) Position in der ersten Detektionseinrichtung die Richtung des Partikelstrahls im Zielvolumen ermittelt werden. Die Position des Partikelstrahls im Zielvolumen kann gleichzeitig der Ort der Entstehung der hochenergetischen Photonen sein.

Die zweite Detektoreinrichtung der Detektorvorrichtung ist bevorzugt ausgelegt und eingerichtet, die Eindringtiefe des Partikelstrahls in dem Zielvolumen mittels der ortsaufgelösten Messung von im Zielvolumen entstehenden Photonen, bevorzugt hochenergetischen Photonen im Energiebereich unterhalb und bis zu einigen MeV zu ermitteln. Da die Zielposition des Partikelstrahls im Zielvolumen, insbesondere im Bragg-Peak der Ort der Entstehung von hochenergetischen Photonen ist, kann mit der Bestimmung des Ortes der Entstehung der Photonen die Lage des Bragg-Peaks des im Zielvolumen applizierten Teilchenstrahls eine Eindringtiefe des Partikelstrahls im Zielvolumen gemessen werden. Hierbei kann der Ort der Entstehung der Photonen mittels der zweiten Detektionseinrichtung ermittelt werden, insbesondere indem die Photonen außerhalb des Zielvolumens ortsaufgelöst nachgewiesen werden. Die Ortsverteilung der das Zielvolumen verlassenden Photonen ist bevorzugt isotrop. Bevorzugt ist die zweite Detektionseinrichtung seitlich von dem Zielvolumen und außerhalb der Strahlachse des Partikelstrahls angeordnet. Besonders bevorzugt ist die zweite Detektionseinrichtung bevorzugt radial um die Strahlachse des Partikelstrahls, insbesondere auf einer Achse angeordnet, die radial vom Zielvolumen nach außen gerichtet ist.

Als zweite Detektionseinrichtung kann hierbei mindestens ein ortsaufgelöst messender Gamma-Detektor eingesetzt werden. Bevorzugt ist die zweite Detektionseinrichtung der Detektorvorrichtung lamellenartig aufgebaut. Die Lamellen der zweiten Detektionseinrichtung sind bevorzugt mit einer Stirnfläche der Lamellen senkrecht zur Partikelstrahlachse angeordnet. Hierbei kann die zweite Detektionseinrichtung auch aus Platten aufgebaut sein, wobei die Platten parallel zueinander angeordnet sind. Die Platten bilden ein so genanntes Plattenstack. Die Platten können mit ihrer schmalen Seite in Richtung des Zielvolumens ausgerichtet sein. Die Erstreckung des Plattenstacks in Richtung der Strahlachse kann bevorzugt größer oder gleich der des Zielvolumens sein. Bevorzugt ist die Stirnseite des Plattenstacks im Wesentlichen parallel zur Strahlachse angeordnet. Die Stirnseiten der einzelnen Platten können hierbei parallel zur Strahlrichtung ausgerichtet sein. Somit können die Photonen aus dem Zielvolumen auf die Stirnseite der einzelnen Platten treffen. Je nach Ort im Zielvolumen treffen die Photonen auf unterschiedliche Platten in einem unterschiedlichen Winkel auf. Die Photonen geben ihre Energie jeweils in einer Platte oder mehrerer Platten ab oder deponieren ihre Energie in der Platte durch die sie hindurch treten. Hierbei werden Elektronen durch verschiedene Prozesse, wie Comptonstreuung, Photoeffekt und Paarbildung erzeugt. Da die Platten oder Lamellen bevorzugt dünn sind, ist eine genügend gute Trennung von unterschiedlichen Entstehungsorten der Photonen im Zielvolumen und damit eine gute Ortsauflösung ermöglicht. Auf Grund des Impulserhaltungssatzes werden die entstehenden Elektronen in der jeweiligen Platte nach vorne gestreut werden. Somit werden diese in Richtung der Fortbewegungsrichtung oder Propagation des ursprünglich entstandenen Photons gestreut. Es können somit nur die Elektronen ihre volle Energie in einer Detektorplatte oder - lamelle abgeben, die senkrecht auf diese treffen. Schräg auftreffende Elektronen werden eine geringere Chance haben, ihre gesamte Energie in einer Platte abzugeben, sondern werden in zwei oder mehreren Platten gemessen werden. Der Die pro Platte gemessene Energie wird kleiner sein für solche Elektronen, als für Elektronen, die ihre Energie in einer Platte abgegeben haben. Somit kann aus der in jeder Platte gemessenen abgegebenen Energie die Richtung der Photonen ermittelt werden und damit der Entstehungsort im Zielvolumen bestimmt werden. Deshalb können die in dem Zielvolumen entstehenden Photonen, insbesondere Gamma-Quanten ortsaufgelöst detektiert werden.

Eine weitere bevorzugte Detektorvorrichtung zur Detektion einer Eindringtiefe eines in ein Zielvolumen applizierten Partikelstrahls zeichnet sich dadurch aus, dass die zweite Detektionseinrichtung als Stoppdetektor ausgebildet ist und derart eingerichtet und ausgelegt ist, dass sie Photonen, die im Zielvolumen entstehen, insbesondere Gamma-Quanten, erfasst, wobei die Detektorvorrichtung weiterhin derart eingerichtet und ausgelegt ist, dass aus dem Abstand zwischen dem Zielvolumen und der zweiten Detektionseinrichtung und der gemessenen Flugzeit der Photonen der Entstehungsort der Photonen ermittelt wird. Die zweite Detektionseinrichtung ist bevorzugt in Strahlrichtung gesehen hinter dem Zielvolumen angeordnet ist. Bevorzugt ist die zweite Detektionseinrichtung auf der Strahlachse oder zumindest symmetrisch zur Strahlachse angeordnet. Als Stoppdetektor können hierbei alle an sich bekannten Plastik-Detektoren eingesetzt werden. Auch ist denkbar, Diamantdetektoren, insbesondere polykristalline Diamantdetektoren einzusetzten. Die Stoppdetektoren sind bevorzugt am distalen Ende des Zielvolumens, insbesondere in Strahlrichtung des Partikelstrahls gesehen hinter dem Zielvolumen angeordnet. Insbesondere haben die Stoppdetektoren eine feste Position. Aus der Position, also dem Abstand vom Zielvolumen zu der zweiter Detektionseinrichtung, und der gemessenen Flugzeit kann der Entstehungsort, insbesondere in z-Richtung der Photonen, insbesondere promptem Gammas ermittelt werden. Als Startsignal für die Flugzeitmessung kann hierbei ein Laserpuls dienen, der die Entstehung des Teilchenstrahls initiiert. Daraus wiederum ist die Position des Partikelstrahls im jeweiligen Rasterpunkt oder Voxel bestimmbar. Es können auch weitere Stopp-Detektoren vorgesehen sein. Insbesondere können die weiteren Stoppdetektoren unter unterschiedlichen Winkeln bezogen auf die Partikelstrahlachse angeordnet sein. Hieraus können durch Rechenalgorithmen die Lage von im Zielvolumen entstehenden Teil-Strahlen rekonstruiert werden.

Die Aufgabe wird ferner durch eine Bestrahlungsvorrichtung zur Bestrahlung eines Zielvolumens in einem Gegenstand gelöst, der mindestens eine Beschleunigervorrichtung aufweist, die eine Strahlerzeugungseinrichtung und eine Strahlformungseinrichtung sowie eine Steuereinrichtung zur Steuerung des Partikelstrahls und mindestens eine erfindungsgemäße Detektorvorrichtung aufweist. Die Beschleunigervorrichtung kann ein an sich bekannter Beschleuniger wie ein Synchrotron oder ein Zyklotron sein, mit dem Partikelstrahlen im Bereich von einigen hundert MeV bis einigen zehn GeV erzeugt werden können. Bei der Beschleunigervorrichtung kann es sich aber bevorzugt auch um einen Laser-induzierten Beschleuniger handeln. Vorteilhaft ist hierbei, dass der Laserstrahl als Startsignal für eine Detektorvorrichtung mit einer als Stoppdetektor eingesetzten zweiten Detektionseinrichtung fungieren kann. Bei einem Laserinduzierten Beschleuniger wird ein Laserstrahl mit sehr hoher Leistung (Peta-Watt-Laser) auf eine Folie geschossen. Hierbei entstehen unter anderem geladene Teilchen, aus denen ein Partikelstrahl geformt werden kann. Dieser kann dann auf ein Zielvolumen geschossen werden und dort deponiert werden, wobei die Partikel auch in einem Bragg-Peak deponiert werden und prompte Gammas durch die Abbremsung entstehen.

In einer bevorzugten Ausgestaltung der Bestrahlungsvorrichtung ist die Detektorvorrichtung derart eingerichtet und ausgebildet, dass diese ein Steuersignal erzeugt, welches in die Steuereinrichtung der Bestrahlungsvorrichtung eingespeist werden kann. Mittels der Steuereinrichtung kann eine Steuerung der Bestrahlung, insbesondere online, bevorzugt gleichzeitig mit der Bestrahlung erfolgen. Insbesondere kann das Steuersignal aus der Detektorvorrichtung die Eindringtiefe des Partikelstrahls betreffend, verwendet werden, um sukzessive Rasterpunkte des Zielvolumens abzuscannen oder abzufahren.

Die Bestrahlungsvorrichtung zeichnet sich dadurch aus, dass die Detektorvorrichtung mindestens eine Detektionseinrichtung zum Nachweis von Photonen, insbesondere hochenergetischen Gammas aufweist. Bevorzugt sind eine erste und/oder mindestens eine zweite Detektionseinrichtung vorgesehen, wobei die erste Detektionseinrichtung und die mindestens zweite Detektionseinrichtung an mindestens zwei unterschiedlichen Positionen relativ zum Zielvolumen bevorzugt in zwei zueinander senkrechten Positionen angeordnet sind. Bevorzugt ist jeweils mindestens die zweite Detektionseinrichtung seitlich von dem Zielvolumen angeordnet. Die zweite Detektionseinrichtung kann insbesondere an einer Position radial um das Zielvolumen angeordnet sein. Hierbei kann diese geodätisch in der Ebene des Zielvolumens oder unterhalb des Zielvolumens angeordnet sein.

Die Aufgabe wird ferner durch ein Verfahren zum Bestimmen einer Eindringtiefe eines Partikelstrahls in einem Gegenstand, insbesondere in einem Zielvolumen des Gegenstandes gelöst, wobei Photonen, insbesondere Gamma-Quanten, welche durch eine Wechselwirkung des Partikelstrahls in dem Gegenstand entstehen, mittels mindestens einer Detektorvorrichtung, insbesondere einer Detektorvorrichtung nach einem der Ansprüche 1 bis 6 erfasst werden.

Bevorzugt kann die Richtung des Partikelstrahls, insbesondere der laterale Ort des Partikelstrahls im Zielvolumen, erfasst werden, insbesondere mit einer ortsaufgelösten Detektionseinrichtung. Hierbei kann die Detektionseinrichtung den Ort des Partikelstrahls im Zielvolumen als Entstehungsort von mindestens einem Photon ermitteln. Dies kann aus einer Messung der in der Detektionseinrichtung gemessenen Energie des Photons bzw. aus diesem entstehenden Elektrons erfolgen.

Bevorzugt können bei dem Verfahren die Photonen, insbesondere Gamma-Quanten an mehreren Positionen außerhalb der Partikelstrahlachse unabhängig voneinander erfasst werden. Hierdurch kann eine lamellenartige Detektionseinrichtung mit dünner Lamellenstärke eingesetzt werden.

Bevorzugt ist das Verfahren derart ausgestaltet, dass der Entstehungsort der Photonen in dem mit dem Partikelstrahl bestrahlten Zielvolumen des Gegenstandes mittels Messung einer Flugzeit der Photonen vom Entstehungsort im Zielvolumen bis zu einer Detektionseinrichtung ermittelt wird. Die Detektionseinrichtung ist hierbei bevorzugt ein Gammadetektor. Die Flugzeitmessung wird hierbei bevorzugt von einem Signal aus der den Teilchenstrahl erzeugenden Laser gestartet.

Dadurch ist das Verfahren geeignet zur Bestimmen eines Ortes eines in ein Zielvolumen applizierten Partikelstrahls, wobei der Ort von in dem Zielvolumen entstehenden Photonen erfasst wird. Hierbei kann insbesondere eine Eindringtiefe eines Partikelstrahls in einen Körper bestimmt werden, wobei insbesondere eine Flugzeit von Gamma-Quanten, welche durch eine Wechselwirkung des Partikelstrahls in dem Zielvolumen entstehen, erfasst werden kann.

Die besonderen technischen Merkmale und Vorteile der vorstehend beschriebenen Vorrichtungen und des Verfahrens werden im Zusammenhang beschrieben und diskutiert. Hierbei sind die Merkmale und Vorteile der Vorrichtungen auf das Verfahren anwendbar und für dieses gültig und umgekehrt.

Im Folgenden wird die Erfindung anhand vorteilhafter Ausführungsbeispiele und unter Bezugnahme auf die beigefügte Zeichnung näher erläutert. Gleich oder ähnliche Gegenstände sind hierbei mit gleichen Bezugszeichen bezeichnet. Es zeigen:
Figur 1: eine Detektorvorrichtung mit einer ersten Detektionseinrichtung und einer zweiten Detektionseinrichtung;
Figur 2: eine lamellenförmige zweite Detektionseinrichtung;
Figur 3a, 3b, 3c, 3d: gemessene Diagramme der zweiten Detektionseinrichtung: Zählrate als Funktion von Energie E_{dep};
Figur 4: Prozesse beim Durchtritt eines Laserstrahls durch eine Folie;
Figur 5: eine Detektorvorrichtung mit einer Anordnung zur Flugzeitmessung;
Figur 6: Diagramm, in dem eine Zählrate von Photonen als Funktion der Photonenenergie aufgetragen ist;
Figur 7: Ablaufdiagramm eines erfindungsgemäßen Verfahrens.

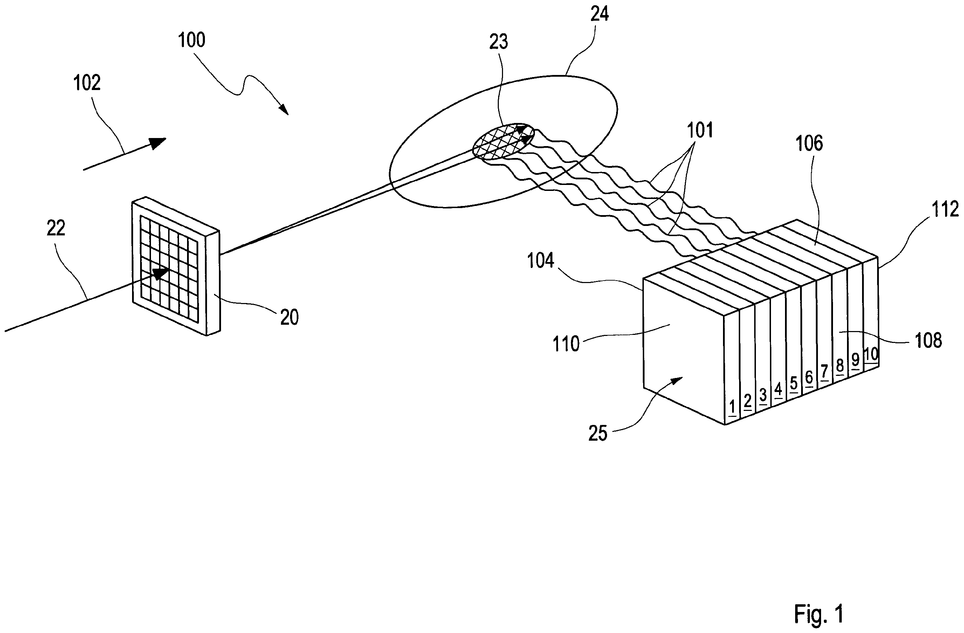

Figur 1 zeigt in schematischer Darstellung eine Detektorvorrichtung 100 mit einer ersten Detektionseinrichtung 20 und einer zweiten Detektionseinrichtung 25. Die erste Detektionseinrichtung 20 ist in Strahlrichtung 102 gesehen, die die Richtung eines Partikelstrahls 22 bezeichnet und mit dem Pfeil 102 dargestellt ist, vor der zweiten Detektionseinrichtung 25 angeordnet. Der Partikelstrahl 22 kann hierbei ein Ionenstrahl sein, insbesondere ein Strahl aus Protonen, Heliumionen, Koh-Ienstoffionen, Neonionen und oder Sauerstoffionen. Der Strahl 22 kann auch Pionen oder Myonen aufweisen, sowie Gemische aus zweien oder mehreren der genannten Partikel.

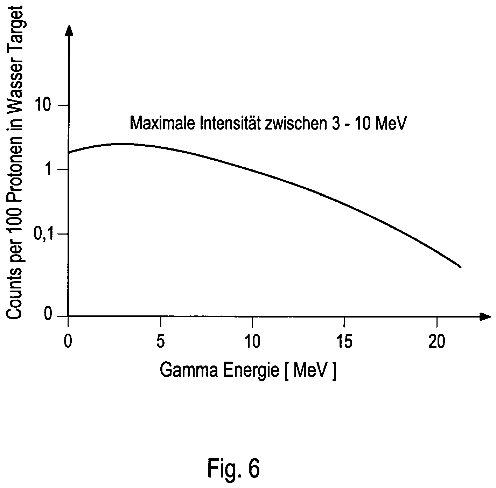

Der Ionenstrahl 22 kann entweder in einer Beschleunigervorrichtung mit einer Ionenquelle erzeugt werden (nicht dargestellt) oder in einer Laserstrahl getrieben Beschleunigervorrichtung. Die Erzeugung des Partikelstrahls 22 ist hierbei unerheblich für die Wirkungsweise der Detektorvorrichtung 100. Die Energie des Partikelstrahls 22 beträgt typischerweise einige 10 MeV/amu bis einige GeV/amu. Hierbei ist die Energie des Partikelstrahls 22 derart gewählt, dass der Partikelstrahl 22 für die Bestrahlung eines Zielvolumens 23 in einem Gegenstand 24, beispielsweise einem Patienten 24 geeignet ist. Der Gegenstand 24, insbesondere der Patient 24 ist hierbei schematisch als Ellipse 24 gezeichnet. Der Gegenstand 24 kann aber auch ein Phantom sein und zur Verifikation von Bestrahlungsparametern vor und nach der Bestrahlung eines Patienten dienen. Hierbei ist die Energie typischerweise derart gewählt, dass der Partikelstrahl 22 einige cm tief in den Gegenstand 24 eindringt und in dem Zielvolumen 23 gestoppt wird. Der Energieverlust des Partikelstrahls 22 entspricht hierbei typischerweise einer Bragg-Verteilung. Im Zielvolumen 23 entstehen Photonen die mit wellenartigen Pfeilen 101 bezeichnet sind. Eine Energieverteilung der entstehenden Photonen ist in Zusammenhang mit Figur 6 näher erläutert. Die Photonen treten aus dem Zielvolumen 23 aus und können außerhalb des Zielvolumens 23 nachgewiesen werden. Die Winkelvertreilung der austretenden Photonen ist typischerweise isotrop. Photonen können in einer oder mehrerer Detektionseinrichtung/Detektionseinrichtungen nachgewiesen werden, die radial um die Strahlachse angeordnet sind. Die entstehenden Photonen werden auch als prompte Gamma-Quanten bezeichnet.

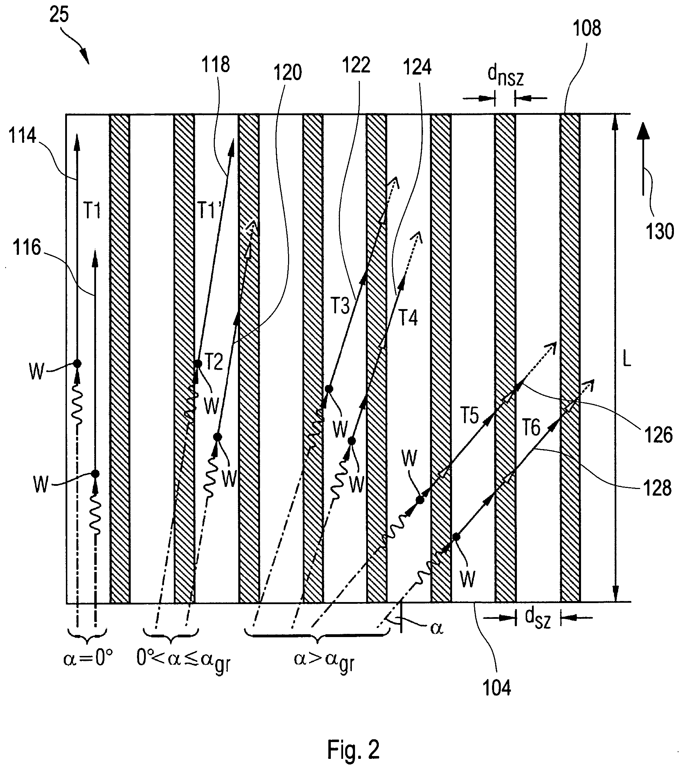

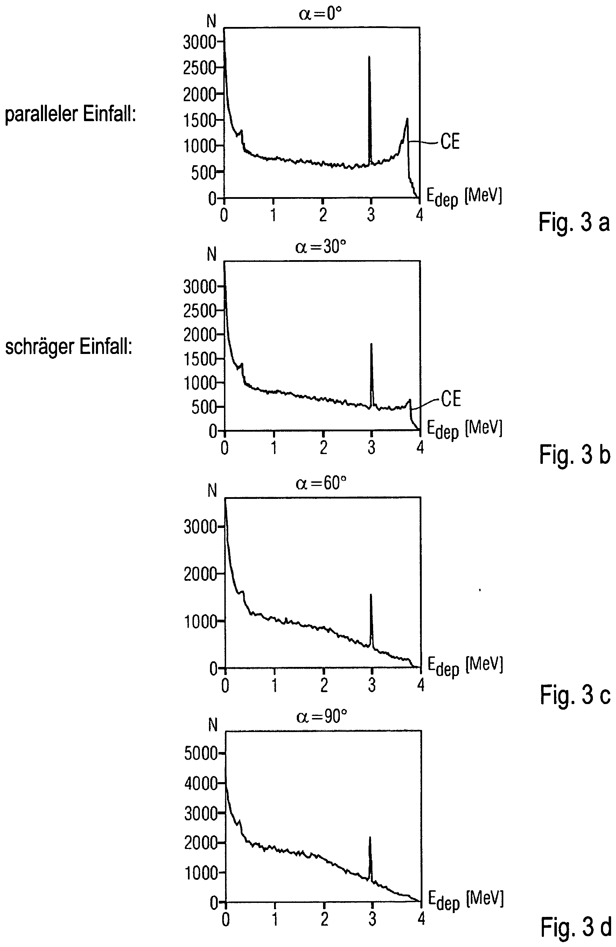

Die zweite Detektionseinrichtung 25 ist als laminarer Detektor aufgebaut und weist Platten oder Lamellen 1, 2, 3, 4, 5, 6,7, 8, 9 und 10 auf. Die Anzahl der Platten 1 bis 10 oder Lamellen 1 bis 10 ist rein beispielhaft zu verstehen und nicht beschränkend. Die Funktionsweise der Detektionseinrichtung 25 erfolgt genauer in der Beschreibung zu den Figuren 2 und 3. Die zweite Detektionseinrichtung 25 ist in schräger Draufsicht gezeigt. Die zweite Detektionseinrichtung 25 weist eine Stirnseite 104 auf, die dem Zielvolumen 23 zugewandt ist. Ferner weist diese eine rückwärtige, dem Zielvolumen 23 abgewandte Rückseite 108 sowie eine erste und eine zweite Seite 110 und 112 sowie eine obere Seite 106 auf.

Die erste Detektionseinrichtung 20 ist bevorzugt ein ortauflösender Detektor oder ein ortsaufgelöst messender Detektor, der einen Durchtrittsort des Partikelstrahls 22 durch diesen erfassen kann. Eine Massenbelegung der Detektionseinrichtung 20 ist hierbei derart gewählt, dass die Detektionseinrichtung 20 ein Transmissionsdetektor ist. Bevorzugt ist die erste Detektionseinrichtung 20 eine Ionisationskammer, eine Drahtkammer oder ein Pixel-Ionisationskammer. Die erste Detektionseinrichtung 20 kann insbesondere eine Pixel-Ionisationskammer mit einer Auflösung von 1812x1707 Pixeln sein. Aber auch Pixel-Ionisationskamern mit kleineren Pixel-Werten sind einsetzbar. Typischerweise kann eine Auflösung kleiner als 3 mm erreicht werden. Typischerweise liegt die Auflösung im µm-Bereich, insbesondere kleiner oder ungefähr gleich 100µm. Typische geometrische Abmessungen der ersten Detektionseinrichtung 20 können ungefähr 40 x 40 cm sein.

Figur 2 zeigt die zweite Detektionseinrichtung 25 von Figur 1 mit einer Anordnung von Platten oder Lamellen 1 bis 10 im Längsschnitt. Der Längsschnitt verläuft parallel zur Stirnseite 104 der zweiten Detektionseinrichtung 25. Mit den Pfeilen 114 und 116 sind die Trajektorien von Photonen und durch diese erzeugten Elektronen bezeichnet, die senkrecht auf die Stirnseite 104 treffen und somit parallel zum Verlauf der Platten "laufen". Die Elektronen können ihre gesamte Energie in einer Platte deponieren, vorausgesetzt diese ist lang genug. Die Pfeile 118 und 120 bezeichnen Trajektorien von Photonen und durch diese erzeugten Elektronen, die unter einem Winkel α₁ auf die Stirnseite 104 der zweiten Detektionseinrichtung 25 treffen. Der Winkel α₁ beträgt hierbei bevorzugt zwischen Null Grad (0°) und einem Winkel α_{gr}. Die Pfeile 122 bis und 128 zeigen eine Trajektorie, die unter einem Winkel α₂ auf die Stirnseite 104 treffen, wobei α₂ größer als α₁ ist. Elektronen, die schräg zur mit dem Pfeil 130 bezeichneten Plattenrichtung verlaufen, haben somit eine kleinere Chance ihre Energie in nur einer Platte abzugeben. Sie werden mehrere Platten durchlaufen und ihre Energie in mehreren Platten abgeben. Somit wird in mehreren Platten ein Energiesignal gemessen werden können, wobei das Energiesignal eine kleinere deponierte Energie zeigen wird. In Figur 3 sind derartige Energiespektren gezeigt. Aus diesen Signalen kann die Reichweite des Partikelstrahls 22 im Zielvolumen 23 ermittelt werden, also der Entstehungsort des Photons im Zielvolumen 23, welches das zugehörige Elektron erzeugt. Die Platten 1 bis 10 werden hierzu einzeln und unabhängig voneinander ausgelesen. Spektren dazu sind in Figur 3 gezeigt.

Bei einer typischen Plattenstärke einer der Platten 1 bis 10 kleiner als 5 mm liegt eine Tiefenauflösung unterhalb von 5mm. Für die Anwendung in der Tumortherapie, also um klinisch relevante Reichweiten des Partikelstrahls 22 im Zielvolumen 23 zu ermitteln, kann mit einer entsprechenden Anordnung und Plattendicke eine Auflösung von typischerweise 1 mm erreicht werden. Hierzu wird bevorzugt eine zweite Detektionseinrichtung 25 seitlich vom Zielvolumen 23 angeordnet und eine weitere Detektionseinrichtung (nicht gezeigt) mit einem in etwas gleichen Aufbau unterhalb des Patienten 24 mit dem Zielvolumen 23. Eine noch kleine Auflösung kann erzielt werden, wenn mehrere Detektionseinrichtungen 25, 25', 25", usw. seitlich und unterhalb des Zielvolumens 23 angeordnet werden. Die Detektionseinrichtung 25 kann prinzipiell auf jeder Position auf einem Kreis um die Strahlachse 102 angeordnet werden.

Die Detektionseinrichtung 25 ist an sich vom Aufbau her in der WO 2008/009528, insbesondere auf den Seiten 23 bis 27 und in den Figuren 3, 4 und 5 näher erläutert, auf die hiermit Bezug genommen wird, wobei die Detektionseinrichtung 25 richtungsempfindlich ist.

Figur 3 zeigt gemessene Energiespektren von in Platten 1 bis 10 der Detektionseinrichtung 25 deponierten Energien von Photonen bzw. entstehenden Elektronen. Die Zählrate N ist als Funktion der Energie E_{dep} in MeV aufgetragen. Hierbei ist die Energie vorzugsweise die in einer Platte der Detektionseinrichtung deponierten Energie der Elektronen. Aufgrund des Impulserhaltungssatzes werden auch die in den Platten der zweiten Detektionseinrichtung 25 durch Compton- oder Photonstreuung oder Paarbildung aus den Photonen entstehenden Elektronen nach vorne, d.h. in Richtung der Propagation der ursprünglich entstehenden Photonen gerichtet sein. In Figur 3a ist ein Energiespektrum für den Fall gezeigt, dass die Photonen parallel zur Anordnung der Platten 1 bis 10 der Detektoreinrichtung 25 auf diese treffen. In Figur 3b ist ein Energiespektrum für einen Winkel α von α= 30°gezeigt. Es ist erkennbar, dass die Anzahl N der gezählten, bzw. registrierten Photonen oder Elektronen bei einer Energie von 3 MeV geringer ist. N beträgt ca. 2000 im Vergleich zu ca. N= 2800 im Spektrum von Figur 3a. Figur 3c zeigt ein Energiespektrum für den Fall von α= 60° in dem die Zählrate N bei einer Energie von 3 MeV kleiner als 2000 Ereignisse gezeigt ist. Figur 3d zeigt ein Energiespektrum aufgenommen für Photonen, bzw. Elektronen unter einem Winkel von α=90°. Hier ist die Anzahl N der registrierten Ereignisse mit einer Energie von 3 MeV noch geringer. Die Spektren in den Figuren 3a, 3b, 3c, 3d sind diejenigen von einzelnen Platten und beispielhaft zu verstehen. Erkennbar ist aus diesen, dass die Photonen bzw. Elektronen, die schräg auf die jeweilige Platte auftreffen weniger Beitrag im hochenergetischen Teil (bei ca. 3 MeV) beitragen können, bzw. dass weniger Ereignisse in der jeweiligen Platte registriert werden. Dies sind in der Regel Platten der Detektionseinrichtung 25, die jenseits der Reichweite von Ionen im Zielvolumen 23 liegen. Dadurch können die Photonen nicht senkrecht auf die Stirnseite 104 der Platte treffen. Typischerweise sind die Platten mit den Nummern 4, 5, 6, 7,8 diejenigen, auf die Photonen senkrecht zur Stirnfläche 104 treffen können. Die Platten mit den Nummern 9 und 10 sowie 1 bis 3 sehen nur Photonen unter schrägem Einfall. Diese Photonen, die auf die Platten 1 bis 3 und 9 und 10 treffen geben weniger Energie in der Platte ab. Die Photonen können nämlich auf Grund der geometrischen Gegebenheiten nicht parallel auf die Platten 1 bis 3 und 9 und 10 der Detektionseinrichtung 25 treffen.

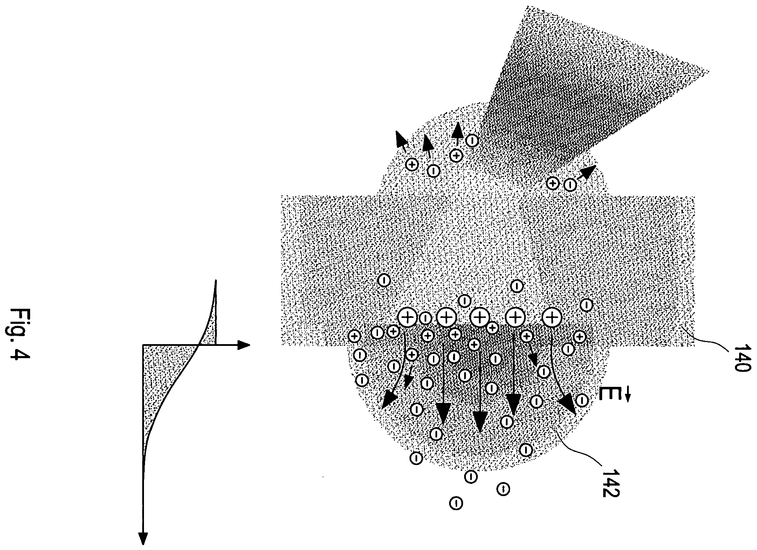

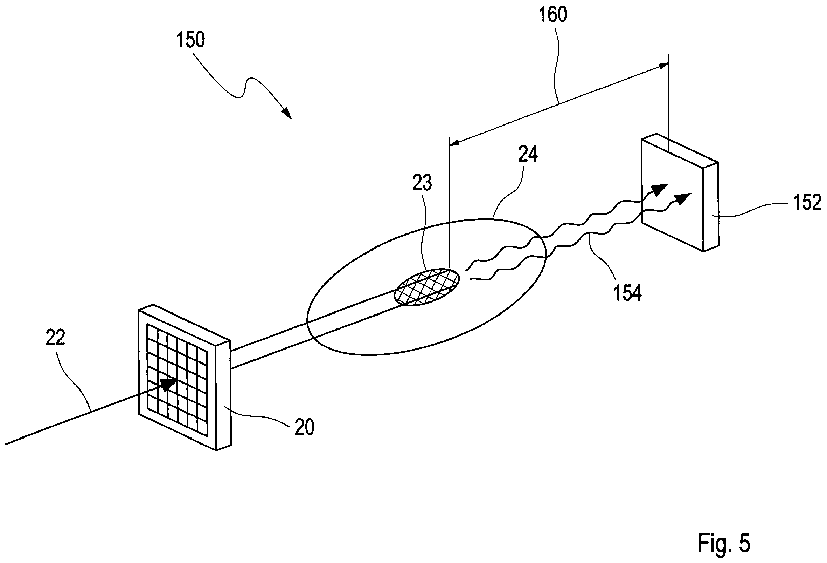

Figur 4 zeigt eine Prinzipskizze der Prozesse die bei der Technik eines lasergetriebenen Beschleunigers eine Rolle spielen. Durch einen Kurzzeitpuls-Laser werden Photonenpulse mit sehr hoher Intensität (vorzugsweise im Bereich Peta-Watt) in einem Zeitfenster von Femtosekunden erzeugt und auf eine dünne Kohlenstofffolie140 geschlossen. In der Folie werden dann zunächst Elektronen (mit "-" bezeichnet) aus einem Atomverband der Kohlenstofffolie 140 freigesetzt, die ein Plasma 142 ausbilden, wobei die übrig gebliebenen Ionen (mit "+" bezeichnet) durch Feldstärken von ungefähr 10¹² Tesla pro Meter in sehr kurzen Beschleunigungsstrecken von weniger als einem Millimeter auf einige MeV beschleunigt werden können. Diese beschleunigten Ionen können einen Partikelstrahl bilden, der zu einem Partikelstrahl, der beispielsweise in den Figuren 1 und 5 mit der Bezugsziffer 22 bezeichnet ist, geformt werden kann. Dieser Partikelstrahl 22 wird zur Bestrahlung des Zielvolumens 23 eingesetzt. Dieser Partikelstrahl 22 oder Ionenstrahl ist in Figur 4 nicht gezeigt.

Figur 5 zeigt in schematischer Darstellung eine Detektorvorrichtung 150. Die Detektorvorrichtung 150 wird bevorzugt an einem lasergetriebenen Beschleuniger mit sehr kurzen Pulsdauern für den Ionenstrahl 22 eingesetzt. Die Detektorvorrichtung 150 weist eine erste Detektionseinrichtung 20 auf. Diese kann beispielsweise eine Vieldrahtkammer sein. Mittels der Detektionseinrichtung 20 kann die Richtung des Partikelstrahls, bzw. dessen laterale Position in x- und y-Richtung bestimmt werden. Ferner weist die Detektorvorrichtung 150 eine zweite Detektionseinrichtung 152 auf. Die zweite Detektionseinrichtung 152 ist in Strahlrichtung des Partikelstrahls 22 gesehen hinter dem Zielvolumen 23 angeordnet und dient als Stoppdetektor. In der zweiten Detektionseinrichtung 152 werden die im Zielvolumen 23 erzeugten Photonen, insbesondere Gammaquanten registriert. Diese können in der zweiten Detektionseinrichtung 152 gestoppt werden, je nach Dicke und Material dieser. Die zweite Detektionseinrichtung 152 ist bevorzugt ein an sich bekannter Plastikdetektor oder ein an sich bekannter Diamantdetektor, bevorzugt aus polykristallinem Diamant. Aus der Position des Stoppdetektors 152, bzw. einer Flugstrecke 160, die sich aus der Entfernung zwischen dem Zielvolumen 23 und der Detektionseinrichtung 152 ergibt, und der gemessenen Flugzeit kann die genaue Position der im Zielvolumen 23 erzeugten Gammastrahlung 154 ermittelt werden. Somit kann die Eindringtiefe des im Zielvolumen 23 applizierten Partikelstrahls 22 ermittelt werden. Neben der zweiten Detektionseinrichtung 152 können auch weitere Stoppdetektoren (nicht gezeigt) unter verschiedenen Winkeln zum Partikelstrahl 22 angeordnet werden.

Die Besonderheiten eines lasergetriebenen Teilchenbeschleunigers und der Zusammenhang zur Detektion der Eindringtiefe im Zielvolumen 23 werden im nachfolgenden generell erläutert. Bei den lasergetriebenen Teilchenbeschleunigern hängt die Zeitschärfe des Teilchenstrahls im Wesentlichen von der Energieschärfe des erzeugten Teilchenpulses ab und wird bei der zukünftigen Applikation dieser Beschleunigungstechnik den nötigen Wert von wenigen Prozenten erreicht haben.

Bisher sind die Laserbeschleunigten Beschleuniger noch in der Erprobungsphase. Realistischerweise kann von einer Zeitschärfe im Bereich weniger Picosekunden ausgegangen. Damit kann festgestellt werden, wann der entsprechend erzeugte Partikelpuls oder Partikelstrahl 22 auf den Körper oder Patienten 24 eingestrahlt wird.

Im Patienten 24 wird bei einer Abbremsstrecke bis zum Zielvolumen 23, der das Tumorgewebe sein kann, die Energie-Aufstreuung und damit die zeitliche Aufstreuung der emittierten prompten Gamma-Quanten sich nur um einige Prozente verbreiten. Man kann also davon ausgehen, dass die prompten Gamma-Quanten ebenfalls in mit einer Pico-Sekundenschärfe emittiert werden. Der Abstand zwischen dem Ort der Gamma-Quanten-Erzeugung und einer prompten Gamma-Detektion kann in eine Flugzeit umgerechnet werden. Das Licht hat eine Geschwindigkeit von 0.3 mm / pico sec. Das bedeutet, dass durch eine Messung der Flugzeit der emittierten Gamma-Quanten in der Detektionseinrichtung 52, also einem Zähler außerhalb des Körpers 24 der Abstand zum Entstehungsort der Photonen auf den Millimeter genau bestimmt werden kann.

Das Detektionsverfahren kann deshalb ein TOF-Verfahren (TOF: Time of Flight) sein, mittels dem die Endringtiefe des (Partikel-)Strahls im Patienten 24 gemessen werden kann. Die Entstehungszeit der prompten Gamma-Quanten ist - wie vorstehend beschrieben durch die Lasergepulste Erzeugung - auf Picosekunden genau bekannt. Dieses Signal kann als Startsignal dienen. In einem schnellen Detektor (zweite Detektionseinrichtung 52, angeordnet auf der Strahlachse 102) außerhalb des Patienten 24, wird das Eintreffen der Gamma-Quanten gemessen und aus der Flugzeit der Abstand zur zweiten Detektionseinrichtung 52, die als StoppDetektor fungiert, ermittelt. Das gemessene Signal resultiert aus allen produzierten Gamma-Quanten unabhängig von der Energie, da die Lichtgeschwindigkeit nicht von der Energie abhängt.

Um die Eindringtiefe zu messen, kann der Stoppdetektor (zweite Detektionseinrichtung 52) in Strahlrichtung auf der distalen Seite des Entstehungsortes der Photonen angeordnet werden. Als Stoppdetektor können die an sich bekannten Plastikdetektoren benutzt werden oder auch zum Beispiel polykristalline Diamantdetektoren. Aus der Messung der Flugzeit der synchron erzeugten Photonen ergibt sich dann die genaue Position des Quellorts/Ursprung der Gamma-Strahlung, also der Ort, an dem der Ionenstrahlen im Zielvolumen 23 beispielsweise des Patienten.

Durch weitere Stopp-Detektoren unter verschiedenen Winkeln zum Primärstrahl (Achse des Ausgangspartikelstrahls) kann dann mit geeigneten Programmen die Lage für jeden Teil-Strahl im Patienten 24 rekonstruiert werden.

In Figur 6 ist in einem Diagramm dargestellt, dass zeigt, dass beim Beschuss eines Wassertargets mit Protonen, Photonen entstehen. In dem Diagramm ist die Anzahl der entstehenden Photonen auf der y-Achse als Funktion von deren Energie auf der x-Achse aufgetragen. Hierbei ist die Anzahl der entstehenden Photonen für Hundert auf ein Wassertarget geschossenen Protonen ermittelt. Die Intensität der entstehenden Photonen liegt zwischen im Wesentlichen 3 bis 10 MeV. Dies entspricht in etwa der Anzahl und der Energie der Photonen, die entstehen, wenn ein Partikelstrahl von Protonen auf ein menschliches Gewebe geschlossen würde. Somit kann dem Diagramm entnommen werden, dass beim Deponieren von Partikelstrahlen in menschlichem Gewebe, Photonen mit einer Energie von ca. 3 bis 10 MeV entstehen. Diese Photonen werden auch als Gamma-Quanten bezeichnet und haben somit ein kontinuierliches Energie Spektrum bis zu einigen MeV und werden isotrop um den im Zielvolumen deponierten Partikelstrahl ausgesendet. Während des Abbremsprozesses der Ionen im Patienten entstehen derartige hoch energetische Gamma-Quanten mit Energien bis zu einigen MeV. Diese Quanten verlassen im Wesentlichen den Patienten und können von außen nachgewiesen werden. Die Anzahl hängt von der Anzahl der deponierten Partikel ab. Die von der zweiten Detektionseinrichtung 25 und 52 nachzuweisenden Photonen können somit diese Energieverteilung ermitteln. Somit wird eine Detektionseinrichtung 25 und/oder 52 derart eingerichtet sein, dass diese Photonen in dem Energiebereich zwischen etwa 3 bis 10 MeV nachweisen können. Somit kann die Detektion von diesen sog. prompten Gamma-Quanten, also elektromagnetischer Strahlung, die während des Abbremsprozesses emittiert wird, zum Nachweis des Ortes dienen, an dem die Partikel des Partikelstrahls 22 im Zielvolumen 23 deponiert werden. Somit wird durch eine Kombination der ersten Detektionseinrichtung 20 und der zweiten Detektionseinrichtung 25 ein richtungsaufgelöster Nachweis erreicht, mit dem die Quelle der Gammastrahlen lokalisiert werden kann. Über die Messung der prompten Gamma-Quanten kann hierbei die Eindringtiefe des Partikelstrahls 22 im Zielvolumen 23 bestimmt werden.

Die Detektorvorrichtung ist hierbei je nach Partikelstrahl unterschiedlich aufgebaut. Im Falle eines kontinuierlichen Strahls, wie dieser mit der Beschleunigereinrichtung, die ein Synchrotron oder Zyklotron aufweist, kann eine Kombination des ortsauflösenden Teilchen-Detektor (Detektionseinrichtung 20) für den Teilchenstrahl 22 vor dem Patienten 24 mit dem laminierten Reichweiten-Gamma-Detektor (zweite Detektionseinrichtung 25) eingesetzt werden, der neben dem Patienten parallel zum Therapiestrahl angebracht ist. Dazu kann ein in der WO 2008/009528 beschriebenes Detektorelement zum Einsatz kommen (vgl. Fig. 1). In einem ortsauflösenden Teilchen Transmissions-Detektor (wie zum Beispiel Drahtkammern, Pixel-Ionisationskammern oder andere ortsauflösende Transmissionszähler geringer Massenbelegung) werden für den Teilchenstrahl eines jeden Voxel-Elements die lateralen (x-y) Koordinaten festgestellt. Durch die gegebene Strahlführung vor dem Patienten oder Körper ist damit auch die Strahlrichtung im Patienten bzw. Körper gegeben.

Die Messung der Eindringtiefe erfolgt, wie bereits in der Beschreibung zu den Figuren 1 und 2 erläutert wurde mit dem laminierten Gamma-Detektor (Detektionseinrichtung 25), wie er insbesondere in den Figuren 1 und 2, sowie Seite 19/Zeile 21 bis Seite 21/Zeile 14 der WO 2008/009528 beschrieben ist. Hierbei, kann diese Tiefe ohne die Verwendung eines Kollimators und damit mit hoher Effizienz bestimmt werden: Für hochenergetische Photonen/Gamma Quanten werden aufgrund des Impulserhaltungssatzes auch die Folgeprodukte, also die im Detektor erzeugten Compton- oder Photo- oder Paarbildungs-Elektronen, nach vorne d.h. in die Richtung der Propagation des ursprünglichen Photons gestreut. Da die Detektionseinrichtung aus einzelnen, insbesondere dünnen Lamellen, d.h. Platten, besteht oder aufgebaut ist, haben nur die Elektronen eine Chance, ihre volle Energie an der Detektionseinrichtung 25 abzugeben, die parallel zu der Platten-Ausrichtung eingetreten sind (vgl. Fig. 2). Elektronen, die schräg zur Platten-Ausrichtung einlaufen, haben eine kleinere Chance ihre Energie in einer Platte zu deponieren. Die ist in der WO 2008/009528 insbesondere auf Seite 23/Zeile 28 bis Seite 27/Zeile 32 zu den dortigen Figuren 3,4 und 5 erläutert, auf die hiermit Bezug genommen wird.

Ähnlich wie in dem bereits beschriebenen Detektionssystem zur Landminensuche aus der WO 2008/009528 sind diese Art von Detektoren richtungs-empfindlich. Im Gegensatz zu dem Landminen-Detektor der WO 2008/009528, bei dem die Richtung einer Quelle lokalisiert wird, kann mit der Detektionseinrichtung 25 eine Reichweite gemessen werden. Deshalb können die Detektormaterialbereiche (Platten aus Detektormaterial) einzeln d.h. unabhängig voneinander ausgelesen werden. Detektoren, die jenseits der Reichweite der Ionen im Patienten liegen, werden nur noch von Gamma-Quanten erreicht, die nicht mehr parallel einfallen können, und deshalb im Hochenergieteil des Energiespektrums wenig beitragen.

Der direkte Nachweis von im Zielvolumen entstehender Photonen bietet Vorteile gegenüber anderen Verfahren, was nachfolgend erläutert wird: Soll eine möglichst zielkonforme Bestrahlung erreicht werden, wird eine aktive Anpassung durchgeführt, die sog. Intensitäts-modulierte Partikel Therapie (IMPT), bei der das Zielvolumen in 20000- 50000 kleine Einzel-Volumina (Voxel) zerlegt wird, die dann nacheinander innerhalb weniger Minuten bestrahlt werden. Für die IMPT wurde als Qualitätssicherung eine zeitgleiche Messung des Positronenzerfalls der im Patienten produzierten Isotope 10 C und 11 C, das sog. in-beam PET, entwickelt und in der Darmstädter Schwerionentherapie angewendet. Mit dieser Technik konnte nach jeder Bestrahlungsfraktion die Reichweiten Verteilung des Strahls im Patienten bestimmt werden und bei einigen % aller Patienten Unstimmigkeiten erkannt und korrigiert werden. Allerdings kann die Analyse mittels PET erst nach der Bestrahlung erfolgen, da die beiden wesentlichen PET Isotope ¹⁰C und ¹¹C Lebensdauern von 19 sec bzw. 20 min haben.

Eine ähnliche Analyse für eine Qualitätssicherung der Dosisdeposition im Patienten wurde auch für die Protonentherapie vorgeschlagen und erprobt, bei der vor allem der Positronenemitter ¹⁵O analysiert wurde (HWZ: 2 min.). Deshalb ist bei den bekannten Verfahren nachteilig, dass diese PET-Analyse zwar bei anderen Ionenprojektilen möglich, jedoch kann auf Grund der jeweiligen Halbwertszeiten immer nur eine posthume Analyse nach jeder Fraktion durchgeführt werden.

Somit kann keine zeitgleiche Beobachtung des Strahls im Patienten erfolgen. Zur Zeit gibt es außer dem In-beam PET, wie es an der Gesellschaft für Schwerionenforschung eingesetzt wurde, kein Standardverfahren, das zur Analyse der prompten Gamma-Quanten, das heißt zur Ortlokalisation des Strahls im Patienten klinisch einsetzbar wäre.

Auch die in der Physik üblichen Energie auflösenden Spektrometer sind dafür ungeeignet, da sie einmal die falsche Informationen liefern: Energie statt Ort und da sie weiter eine geringe Effizienz (Ansprechwahrscheinlichkeit nur im % Bereich) haben, und da diese Effizienz mit der Gamma Energie im Allgemeinen abnimmt und für den MeV Bereich gering ist.

Szintillations-Gamma-Detektoren, wie NaJ oder Plastik-Szintillatoren, oder andere Gamma-Detektoren (Si- oder Ge- Halbleiter) haben per se keine Richtungs-Auflösung. Diese könnte durch einen vorgeschalteten Kollimator erreicht werden aber wieder nur auf Kosten der Effizienz. Auch Kollimator-freie Compton-Detektor Anordnungen, bei denen in einer ersten Streu-Folie das Photon zur Emission eines Elektrons aus der Folie gebracht und dann aus der Analyse der gesamten Kinetik der Entstehungsort bzw. die Richtung des primären Photons/Gamma-Quants bestimmt wird können nicht sinnvoll eingesetzt werden. Dies deshalb, da auch Compton-Zähler eine kleine Effizienz von wenigen Prozenten haben und deshalb kaum geeignet für den klinischen Gebrauch sind.

Deshalb sind die erfindungsgemäße Detektorvorrichtung und das Verfahren, welche die prompt emittierten Gamma-Quanten messen, vorteilhaft. Die Gamma-Quanten haben hierbei je nach der Zeitstruktur des Therapiestrahls, d.h. je nachdem ob der Ionen-Strahl kontinuierlich (oder quasi-kontinuierlich) wie beim Synchrotron, oder ob der Strahl in Mikropulsen, wie bei den z. Zt. entwickelten lasergetriebenen Beschleunigern emittiert wird, eine andere Zeitstruktur. (Das Zyklotron steht mit seiner Zeitstruktur den gepulsten Beschleunigertypen näher).

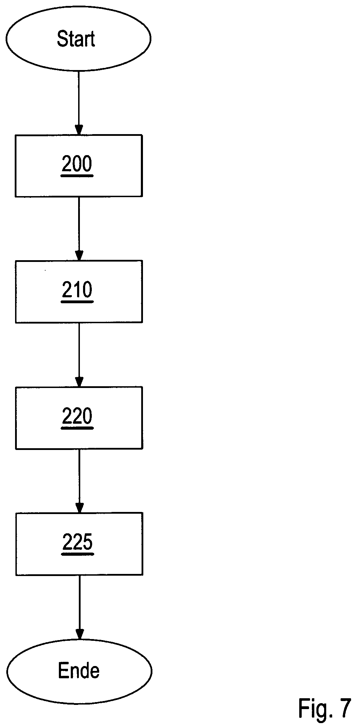

In Figur 7 ist ein Verfahren dargestellt mit dem die Eindringtiefe eines Partikelstrahls 22 in einem zu bestrahlenden Zielvolumen 23 eines Gegenstandes 24 ermittelt werden kann. Im Verfahrensschritt 200 wird die Richtung des Partikelstrahls ermittelt. Dies kann bevorzugt mit einer ersten Detektionseinrichtung 20 erfolgen, die eine mindestens eine laterale Position des Partikelstrahls 22 bestimmt. In einem zweiten Verfahrensschritt 210 wird mit einer ortsauflösenden Detektionseinrichtung die z-Position von durch den Partikelstrahl 22 im Zielvolumen 23 entstehenden Photonen ermittelt. Im Verfahrensschritt 220 wird die ermittelte x-/bzw. y-Position des Partikelstrahls 22 im Zielvolumen 23 und die ermittelte z-Position kombiniert und ein bestrahlter Rasterpunkt im Zielvolumen ermittelt. Im Verfahrensschritt 225 kann ein Steuersignal ausgegeben werden. Das Steuersignal kann die eine Steuerungseinrichtung zur Steuerung der Bestrahlungsvorrichtung und/oder der Beschleunigereinrichtung eingespeist werden.

Der Verfahrensschritt 210 kann in einer Ausgestaltung des Verfahrens alternativ durch eine Messung einer Flugzeit der nach vorne, also in Strahlrichtung gesehen emittierten Photonen erfolgen. Der Abstand der zwischen der als Stoppdetektor für die Photonen dienenden zweiten Detektionseinrichtung und dem Erzeugungsort der Photonen im Zielvolumen durch den Partikelstrahl wird hierbei über die Messung der Flugzeit der Photonen ermittelt. Somit kann der genaue Entstehungsort der Photonen im Zielvolumen ermittelt werden. Die zweite Detektionseinrichtung 52 ist hierbei unter im Wesentlichen Null Grad, bevorzugt auf der Strahlachse 102 angeordnet. Somit kann die Eindringtiefe des Partikelstrahls 22 in dem Zielvolumen 23 ermittelt werden.

## Patentansprüche

1. Detektorvorrichtung zur Detektion einer Eindringtiefe eines in ein Zielvolumen (23) applizierten Partikelstrahls (22) mit einer ersten Detektionseinrichtung (20) für den Partikelstrahl (22) und einer zweiten Detektionseinrichtung (25, 152), wobei die zweite Detektionseinrichtung (25, 152) eingerichtet und ausgelegt ist, Photonen (101, 154), die im Zielvolumen entstehen, insbesondere Gamma-Quanten, zu erfassen, **dadurch gekennzeichnet, dass** die zweite Detektionseinrichtung (25, 152) in Strahlrichtung des Partikelstrahls (22) gesehen hinter der ersten Detektionseinrichtung (20) angeordnet ist, und die erste Detektionseinrichtung (20) derart eingerichtet und ausgelegt ist, dass mittels der ersten Detektionseinrichtung (20) eine Richtung des Partikelstrahls (22) in einer ersten lateralen Dimension sowie in einer zweiten, von der ersten verschiedenen lateralen Dimension bestimmbar ist.

2. Detektorvorrichtung nach Anspruch 1, **dadurch gekennzeichnet, dass** die erste Detektionseinrichtung (20) eine Ionisationskammer oder eine Drahtkammer ist.

3. Detektorvorrichtung nach einem der Ansprüche 1 oder 2, **dadurch gekennzeichnet, dass** die zweite Detektionseinrichtung (25, 152) ausgelegt und eingerichtet ist, die in dem Zielvolumen (23) entstehenden Photonen (101, 154), insbesondere Gamma-Quanten ortsaufgelöst zu detektieren.

4. Detektorvorrichtung nach einem der Ansprüche 1 bis 3, **dadurch gekennzeichnet, dass** die zweite Detektionseinrichtung (25) lamellenartig aufgebaut ist.

5. Detektorvorrichtung nach Anspruch 4, **dadurch gekennzeichnet, dass** die Lamellen der zweiten Detektionseinrichtung (25) mit einer Stirnfläche (104) der Lamellen (1, 2, 3, 4, 5, 6, 7, 8, 9, 10) senkrecht zur Partikelstrahlachse (102) angeordnet sind.

6. Detektorvorrichtung nach einem der Ansprüche 1 bis 5, bei der die zweite Detektionseinrichtung (152) als Stoppdetektor ausgebildet ist und derart eingerichtet und ausgelegt ist, Photonen (101, 154), die im Zielvolumen (23) entstehen, insbesondere Gamma-Quanten (101, 154) zu erfassen, wobei die Detektorvorrichtung weiterhin derart eingerichtet und ausgelegt ist, dass aus dem Abstand zwischen dem Zielvolumen (23) und der zweiten Detektionseinrichtung (152) und der gemessenen Flugzeit der Photonen (101, 154) der Entstehungsort der Photonen (101, 154) ermittelt wird.

7. Bestrahlungsvorrichtung zur Bestrahlung eines Zielvolumens (23) in einem Gegenstand (24), aufweisend eine Beschleunigervorrichtung und eine Steuereinrichtung zur Steuerung des Partikelstrahls (22) und eine Detektorvorrichtung (100, 150) gemäß einem der Ansprüche 1 bis 6.

8. Bestrahlungsvorrichtung gemäß Anspruch 7, wobei die Detektorvorrichtung (100, 150) derart eingerichtet und ausgebildet ist, dass diese ein Steuersignal erzeugt, welches in die Steuereinrichtung der Bestrahlungsvorrichtung eingespeist werden kann.

9. Bestrahlungsvorrichtung gemäß einem der Ansprüche 7 oder 8, **dadurch gekennzeichnet, dass** die erste Detektionseinrichtung (20) und die zweite Detektionseinrichtung (25, 152) an mindestens zwei unterschiedlichen, zueinander senkrechten Positionen relativ zu dem Zielvolumen (23) angeordnet sind.

10. Verfahren zum Bestimmen einer Eindringtiefe eines Partikelstrahls (22) in einem Gegenstand (24), insbesondere in einem Zielvolumen (23) des Gegenstandes (24), das Verfahren umfassend:
Erfassen von Photonen (101, 154), insbesondere Gamma-Quanten, welche durch eine Wechselwirkung des Partikelstrahls (22) in dem Gegenstand (24) entstehen, mittels einer Detektorvorrichtung (100, 150) nach einem der Ansprüche 1 bis 6.

11. Verfahren nach Anspruch 10, zusätzlich umfassend:
Bestimmen der Richtung des Partikelstrahls (22), insbesondere des lateralen Orts des Partikelstrahls (22) im Zielvolumen (23), insbesondere mit einer ortsaufgelösten Detektionseinrichtung (20).

12. Verfahren nach Anspruch 10 oder 11, zusätzlich umfassend:
Erfassen der Photonen, insbesondere Gamma-Quanten, an mehreren Positionen außerhalb der Partikelstrahlachse (102) unabhängig voneinander

13. Verfahren nach einem der Ansprüche 10 bis 12, zusätzlich umfassend:
Ermitteln des Entstehungsorts der Photonen in dem mit dem Partikelstrahl (22) bestrahlten Zielvolumen (23) des Gegenstandes (24) mittels Messung einer Flugzeit der Photonen vom Entstehungsort im Zielvolumen (23) bis zu einer Detektionseinrichtung (152).

## Claims

1. Detector device for detecting a penetration depth of a particle beam (22) applied into a target volume (23), having a first detection unit (20) for said particle beam (22) and a second detection unit (25, 152) where said second detection unit (25, 152) is set up and designed to record photons (101, 154) produced in the target volume, in particular gamma quanta, **characterized in that** said second detection unit (25, 152) is, when viewed in the beam direction of said particle beam (22), arranged behind said first detection unit (20), and said first detection unit (20) is set up and designed such that by means of said first detection unit (20) a direction of said particle beam (22) is determinable in a first lateral dimension as well as in a second lateral dimension differing from the first one.

2. Detector device according to claim 1, **characterized in that** the first detection unit (20) is an ionization chamber or a wire chamber.

3. Detector device according to one of claims 1 or 2, **characterized in that** the second detection unit (25, 152) is designed and set up to detect with local resolution the photons (101, 154) produced in the target volume (23), in particular gamma quanta.

4. Detector device according to one of claims 1 to 3, **characterized in that** the second detection unit (25) is constructed in lamellar form.

5. Detector device according to claim 4, **characterized in that** the lamellas of the second detection unit (25) are arranged with a front face (104) of said lamellas (1, 2, 3, 4, 5, 6, 7, 8, 9, 10) perpendicular to the particle beam axis (102).

6. Detector device according to one of claims 1 to 5, in which the second detection unit (152) is designed as a stop detector and is set up and designed to record photons (101, 154) produced in the target volume, in particular gamma quanta (101, 154), where said detector device is furthermore set up and designed such that the point of origin of said photons (101, 154) is ascertained from the distance between the target volume (23) and said second detection unit (152) and the measured flight time of said photons (101, 154).

7. Irradiation device for irradiating a target volume (23) in an object (24), having an accelerator device and a control unit for controlling the particle beam (22) and a detector device (100, 150) according to one of claims 1 to 6.

8. Irradiation device according to claim 7, wherein the detector device (100, 150) is set up and designed such that it generates a control signal which can be fed into the control unit of said irradiation device.

9. Irradiation device according to one of claims 7 or 8, **characterized in that** the first detection unit (20) and the second detection unit (25, 152) are arranged relative to the target volume (23) at at least two different positions perpendicular to one another.

10. Method for determining a penetration depth of a particle beam (22) in an object (24), in particular in a target volume (23) of said object (24), said method comprising:
recording of photons (101, 154), in particular gamma quanta, produced by an interaction of said particle beam (22) in the object, by means of a detector device (100, 150) according to one of claims 1 to 6.

11. Method according to claim 10, additionally comprising:
determination of the direction of the particle beam (22), in particular of the lateral place of said particle beam (22) in the target volume (23), in particular with a locally resolved detection unit (20).

12. Method according to claim 10 or 11, additionally comprising:
recording of photons, in particular gamma quanta, at several positions outside the particle beam axis (102) independently of one another.

13. Method according to one of claims 10 to 12, additionally comprising:
ascertaining of the point of origin of the photons in the target volume (23) of the object (24) irradiated with the particle beam (22) by means of measurement of a flight time of said photons from their point of origin in said target volume (23) to a detection unit (152).

## Revendications

1. Détecteur servant à détecter une profondeur de pénétration d'un faisceau de particules (22) appliqué dans un volume cible (23), doté d'un premier équipement de détection (20) pour le faisceau de particules (22) et d'un second équipement de détection (25, 152), sachant que le second équipement de détection (25, 152) est configuré et conçu pour acquérir des photons (101, 154) qui sont créés dans le volume cible, notamment des quanta gamma, **caractérisé en ce que** le second équipement de détection (25, 152) est disposé derrière le premier équipement de détection (20), vu dans le sens du faisceau de particules (22), et que le premier équipement de détection (20) est configuré et conçu de telle sorte qu'il est possible au moyen du premier équipement de détection (20) de déterminer une direction du faisceau de particules (22) dans une première dimension latérale, ainsi que dans une seconde dimension latérale différente de la première.

2. Détecteur selon la revendication 1, **caractérisé en ce que** le premier équipement de détection (20) est une chambre d'ionisation ou une chambre à fils.

3. Détecteur selon une des revendications 1 ou 2, **caractérisé en ce que** le second équipement de détection (25, 152) est conçu et configuré pour la détection à résolution spatiale des photons (101, 154), notamment des quanta gamma, créés dans le volume cible (23).

4. Détecteur selon une des revendications 1 à 3, **caractérisé en ce que** le second équipement de détection (25) est constitué de lamelles.

5. Détecteur selon la revendication 4, **caractérisé en ce que** les lamelles du second équipement de détection (25) sont disposées avec une face frontale (104) desdites lamelles (1, 2, 3, 4, 5, 6, 7, 8, 9, 10) perpendiculaire à l'axe du faisceau de particules (102).

6. Détecteur selon une des revendications 1 à 5, dans lequel le second équipement de détection (152) est constitué sous la forme d'un détecteur d'arrêt et configuré et conçu de sorte à acquérir les photons (101, 154) qui se créent dans le volume cible, notamment les quanta gamma (101, 154), sachant que le détecteur est également configuré et conçu de telle sorte que le lieu d'apparition des photons (101, 154) est déterminé à partir de la distance entre le volume cible (23) et le second équipement de détection (152) et le temps de propagation mesuré des photons (101, 154).

7. Dispositif d'irradiation pour irradier un volume cible (23) dans un objet (24), comprenant un accélérateur et une installation de commande servant à commander le faisceau de particules (22), ainsi qu'un détecteur (100, 150) selon une des revendications 1 à 6.

8. Dispositif d'irradiation selon la revendication 7, sachant que le détecteur (100, 150) est configuré et conçu de sorte à produire un signal de commande qui peut être fourni à l'installation de commande du dispositif d'irradiation.

9. Dispositif d'irradiation selon une des revendications 7 ou 8, **caractérisé en ce que** le premier équipement de détection (20) et le second équipement de détection (25, 152) sont disposés, par rapport au volume cible (23), à au moins deux positions différentes perpendiculaires entre elles.

10. Procédé pour déterminer une profondeur de pénétration d'un faisceau de particules (22) dans un objet (24), notamment dans un volume cible (23) de l'objet (24), ledit procédé comprenant :
l'acquisition des photons (101, 154), notamment des quanta gamma qui sont créés par interaction du faisceau de particules (22) dans l'objet, au moyen d'un détecteur (100, 150) selon une des revendications 1 à 6.

11. Procédé selon la revendication 10, comprenant en outre :
la détermination de la direction du faisceau de particules (22), notamment de la position latérale du faisceau de particules (22) dans le volume cible (23), notamment avec un équipement de détection à résolution spatiale (20).

12. Procédé selon la revendication 10 ou 11, comprenant en outre :
l'acquisition des photons, notamment des quanta gamma, en plusieurs positions à l'extérieur de l'axe du faisceau de particules (102) et indépendantes les unes des autres.

13. Procédé selon une des revendications 10 à 12, comprenant en outre :
la détermination du lieu d'apparition des photons dans le volume cible (23) de l'objet (24) irradié avec le faisceau de particules (22) en mesurant un temps de propagation des photons de leur lieu d'apparition dans le volume cible (23) jusqu'à un équipement de détection (152).
